# FASCICULE DE BREVET EUROPEEN

(11) **EP 3 043 729 B1**
(45) Date de publication et mention de la délivrance du brevet: **02.08.2017**
(21) Numéro de dépôt: 14796534.7
(22) Date de dépôt: 09.09.2014
(51) Int. Cl.: A61B 17/74, A61B 17/80

(54) **PLAQUE POUR OSTEOSYNTHESE DE L'EXTREMITE SUPERIEURE DU FEMUR**
PLATTE ZUR OSTEOSYNTHESE DES OBERSCHENKELS
PLATE FOR OSTEOSYNTHESIS OF THE TOP END OF THE FEMUR

(30) Priorité: 10.09.2013 FR 1358673
(43) Date de publication de la demande: 20.07.2016
(73) Titulaire: D.L.P., 44115 Haute Goulaine (FR)
(72) Inventeur: LEFEVRE, Christian, F-29200 Brest (FR); GERARD, Romain, F-29200 Brest (FR); LARCHE, Grégoire, F-49300 Cholet (FR); PODGORSKI, Jean-Pierre, F-49230 St-crespin Sur Moine (FR)
(74) Mandataire: Jacobacci Coralis Harle
(86) Numéro de dépôt international: PCT/FR2014/052229
(87) Numéro de publication internationale: WO 2015/036686

(56) Documents cités:
- WO-A1-2008/019511
- WO-A1-2010/061410
- DE-U1- 20 109 273
- US-A1- 2009 312 758

## Description

### DOMAINE TECHNIQUE AUQUEL SE RAPPORTE L'INVENTION

La présente invention concerne le domaine du matériel médical pour technique d'ostéosynthèse.

L'invention concerne plus précisément une plaque pour ostéosynthèse de l'extrémité supérieure d'un fémur, en particulier pour la réduction de fractures survenant au niveau de son grand trochanter.

### ARRIERE-PLAN TECHNOLOGIQUE

Chez l'homme, l'extrémité proximale (ou supérieure) du fémur comporte une structure anatomique dénommée « trochanter » et formée plus précisément de deux excroissances ou bossages, l'une appelée petit trochanter et l'autre grand trochanter, situées de chaque côté de l'os près de l'articulation pelvienne.

Le grand trochanter (trochanter major) est une éminence quadrilatérale du fémur située à la jonction de son col avec la partie supérieure de son corps.

Cette structure anatomique comporte deux surfaces et quatre arêtes qui sont impliquées dans l'insertion de muscles et de ligaments :
- la surface latérale sert d'insertion au muscle moyen glutéal (gluteus médius) ;
- la surface médiale présente à sa base une dépression, la fosse trochantérique (fossa trochanterica), où s'insère le tendon du muscle obturateur externe (obturator externus) ; au-dessus et en avant se trouvent le lieu de l'attachement de l'obturateur interne et des muscles jumeaux ;
- l'arête supérieure est le lieu d'insertion du muscle piriforme ;
- l'arête inférieure donne naissance à la partie supérieure du vaste latéral (vastus lateralis) ;
- l'arête antérieure est proéminente, le muscle petit glutéal (gluteus minimus) s'y rattachant.

Les fractures du grand trochanter sont généralement traitées par un dispositif d'ostéosynthèse constitué d'une plaque maintenue fixe, en appui contre la face extérieure du fémur dont elle épouse le profil, grâce à des vis corticales pénétrant dans le matériau osseux de réception (voir par exemple la plaque pour ostéosynthèse décrite dans le document US - 2009/312758).

L'extrémité supérieure de cette plaque d'ostéosynthèse comporte généralement deux branches formant crochets, qui sont recourbées pour venir recouvrir le grand trochanter et dont l'extrémité acérée vient s'impacter dans l'os.

Les indications d'un tel dispositif d'ostéosynthèse sont notamment (i) la fixation du volet de fémorotomie, (ii) une complémentation d'ostéosynthèse de certaines fractures sur tige

Les indications d'un tel dispositif d'ostéosynthèse sont notamment (i) la fixation du volet de fémorotomie, (ii) une complémentation d'ostéosynthèse de certaines fractures sur tige prothétique fémorale et des fractures per- et sous-trochantérienne du sujet âgé, ou (iii) les fractures ou pseudarthroses du grand trochanter.

Mais en pratique, il s'avère que les plaques d'ostéosynthèse actuelles ne permettent pas une gestion optimale des forces d'arrachement en traction des muscles, ni une cohésion optimale muscle-os-plaque au niveau de l'extrémité supérieure du fémur.

De plus, le positionnement purement latéral du crochet trochantérien des plaques actuelles crée une incapacité à maintenir le foyer de fracture en compression sous l'effet des efforts musculaires, qui s'exercent en zone postérieure. L'absence de l'effet dit « hauban » conduit à des ruptures du matériel d'ostéosynthèse, car soumis à de trop fortes contraintes en flexion.

Dans ce contexte, il existe un besoin pour des plaques d'ostéosynthèse apportant une solution optimale à ces contraintes.

### OBJET DE L'INVENTION

Les demandeurs ont constaté que, de manière surprenante, les limites actuelles proviennent de l'agencement des crochets ménagés à l'extrémité de la plaque d'ostéosynthèse.

Dans ce contexte, les demandeurs ont développé une nouvelle structure de plaque d'ostéosynthèse qui vise à offrir une gestion efficace des efforts de compression par une utilisation des efforts musculaires entraînant une mise en compression du foyer d'ostéosynthèse (effet « hauban »), condition indispensable à la mise en place du processus de consolidation osseuse.

Une telle structure permet, en pratique, une conformation efficace et stable des fragments entre eux, et diminue les efforts supportés par la plaque d'ostéosynthèse (en s'opposant à tout phénomène d'ouverture et de bâillement du foyer de fracture).

Pour cela, la plaque d'ostéosynthèse selon l'invention comporte deux parties :
- une partie longitudinale dont une face de dessous est destinée à recouvrir la face externe du corps du fémur, laquelle partie longitudinale définit un axe longitudinal et est munie de plusieurs orifices traversants pour le positionnement d'éléments de fixation dans le matériau osseux de réception, et
- une partie d'extrémité supérieure dont une face de dessous est destinée à se positionner sur le grand trochanter du fémur, laquelle partie d'extrémité supérieure comporte au moins deux branches formant crochets qui sont recourbées du côté de la face de dessous de ladite partie longitudinale inférieure et qui comportent chacune un plan médian défini par leur ligne courbe médiane ;
et ces branches sont agencées de sorte que :
(i) le plan médian d'une première desdites branches définit un angle maximal de 15° par rapport à l'axe longitudinal de ladite partie longitudinale, et
(ii) le plan médian d'une seconde desdites branches définit un angle compris entre 50° et 110° par rapport à l'axe longitudinal de ladite partie longitudinale.

La première desdites branches est ainsi agencée de sorte à recouvrir la partie latérale du grand trochanter et la seconde desdites branches est destinée à recouvrir la partie postérieure du grand trochanter.

Les performances de cette plaque d'ostéosynthèse sont en particulier liées à cette seconde branche qui est largement désaxée par rapport à l'agencement rencontré dans les plaques actuelles.

Cet agencement de la seconde branche vise en particulier à exercer une compression du foyer de fracture par effet « hauban », lors de la mise en mouvement d'avant en arrière du fémur autour de l'articulation de la hanche (passage de la position assise à la position debout).

Selon un mode de réalisation préféré, les deux branches sont agencées de sorte que :
(i) le plan médian de la première desdites branches définit un angle maximal de 15° par rapport à l'axe longitudinal de ladite partie longitudinale, et
(ii) le plan médian de la seconde desdites branches définit un angle compris entre 70° et 90° par rapport à l'axe longitudinal de la partie longitudinale de la plaque.

D'autres caractéristiques avantageuses, pouvant être prises en combinaison ou indépendamment les unes des autres, sont indiquées ci-dessous :
- les plans médians respectifs des deux branches s'étendent perpendiculairement, ou au moins approximativement perpendiculairement, par rapport à un plan général passant par la face de dessous de la partie longitudinale de la plaque ;
- les branches présentent chacune deux bordures longitudinales, à savoir : une bordure intérieure en regard de l'autre branche et une bordure extérieure opposée ; et par rapport à la bordure longitudinale extérieure de la première branche : la bordure longitudinale extérieure de la seconde branche définit un angle compris entre 80° et 100°, et la bordure longitudinale intérieure de la seconde branche définit un angle compris entre 60° et 80° ;
- la partie d'extrémité supérieure de la plaque comporte deux bras qui sont, d'un côté inférieur, raccordés à la partie longitudinale et, d'un côté supérieur, raccordés par une traverse et prolongés chacun par l'une des branches formant crochets.

Selon un mode de réalisation particulièrement intéressant, la partie longitudinale et la partie d'extrémité supérieure de la plaque sont dissociables, avec des moyens pour leur solidarisation amovible l'une avec l'autre.

Dans ce cas, la partie longitudinale et la partie d'extrémité supérieure comportent avantageusement, pour l'une, un tronçon saillant et, pour l'autre, une empreinte complémentaire ; lequel tronçon saillant et laquelle empreinte complémentaire comportent des orifices traversants destinés à être alignés pour la réception d'éléments rapportés de solidarisation entre eux, tels que des vis par exemple.

Encore dans ce cas, le tronçon saillant et l'empreinte complémentaire sont avantageusement tous deux délimités latéralement par deux bordures longitudinales en regard définissant un retreint local, pour assurer un verrouillage mécanique en translation selon l'axe longitudinal de la partie longitudinale de la plaque.

De même, le tronçon saillant et l'empreinte complémentaire comportent avantageusement deux orifices traversants pour le positionnement des éléments de solidarisation précités, ménagés de part et d'autre dudit retreint local.

Encore dans ce cas, la partie longitudinale ou la partie d'extrémité supérieure, munie de l'empreinte, comporte deux orifices traversants ménagés de part et d'autre du retreint local, pour le positionnement d'éléments de fixation dans le matériau osseux de réception.

De préférence, la partie d'extrémité supérieure comporte le tronçon saillant, et la partie longitudinale comporte l'empreinte complémentaire.

La présente invention concerne encore le dispositif d'ostéosynthèse comprenant la plaque d'ostéosynthèse précisée ci-dessus, en combinaison avec les éléments (par exemple des vis) pour la fixation de cette plaque d'ostéosynthèse sur le matériau osseux de réception.

### DESCRIPTION DETAILLEE DE L'INVENTION

La présente invention sera encore illustrée, sans être aucunement limitée, par la description suivante d'un mode de réalisation particulier, en relation avec les dessins annexés dans lesquels :
- la figure 1 est une vue générale de la plaque d'ostéosynthèse, orientée du côté de sa face de dessus ;
- la figure 2 est une vue de détails et agrandie de la plaque d'ostéosynthèse selon la figure 1, au niveau de sa partie d'extrémité supérieure destinée à recouvrir le grand trochanter du fémur ;
- la figure 3 correspond à la vue de détails et agrandie de la plaque d'ostéosynthèse selon la figure 2, vue ici du côté de sa face de dessous ;
- la figure 4 représente, en perspective et du côté de sa face de dessus, la partie d'extrémité supérieure constitutive de la plaque d'ostéosynthèse, dissociée de la partie longitudinale ;
- la figure 5 montre encore la partie d'extrémité supérieure de la plaque d'ostéosynthèse, selon une autre perspective orientée du côté de ses deux branches formant crochets ;
- la figure 6 représente l'extrémité supérieure de la partie longitudinale de la plaque d'ostéosynthèse, munie d'une empreinte destinée à recevoir la partie d'extrémité supérieure amovible ;
- la figure 7 illustre la plaque d'ostéosynthèse selon les figures 1 à 3, vue du côté de sa face de dessus et convenablement rapportée sur un fémur gauche ;
- la figure 8 est une vue similaire à la figure 7, représentant la partie supérieure de la plaque d'ostéosynthèse avec l'os associé, observés ici sur la face antérieure de l'os ;
- la figure 9 illustre encore la plaque d'ostéosynthèse et le fémur associé, avec ladite plaque d'ostéosynthèse observée ici selon l'axe tête/col du fémur.

La plaque d'ostéosynthèse 1 illustrée sur les figures est dédiée à la réduction de fractures touchant le grand trochanter.

Il est rappelé que le grand trochanter constitue une partie de l'os du fémur F.

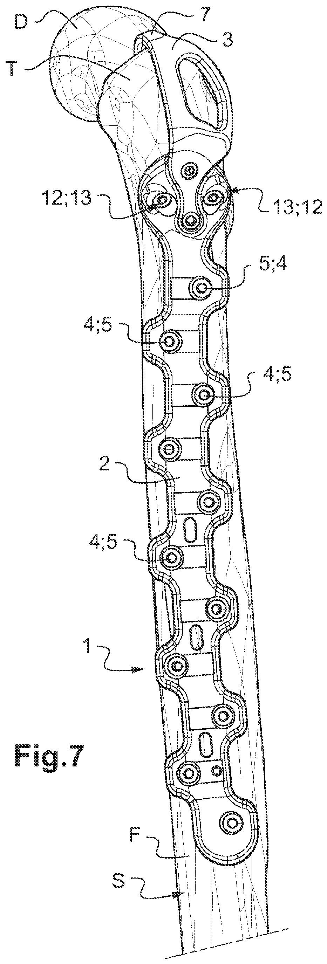

En particulier, tel qu'illustré sur les figures 7 à 9, la partie proximale (supérieure) du fémur F comporte le grand trochanter T qui constitue une éminence quadrilatérale située à la jonction entre le col du fémur C terminée par la tête du fémur D et la partie supérieure du corps de fémur S.

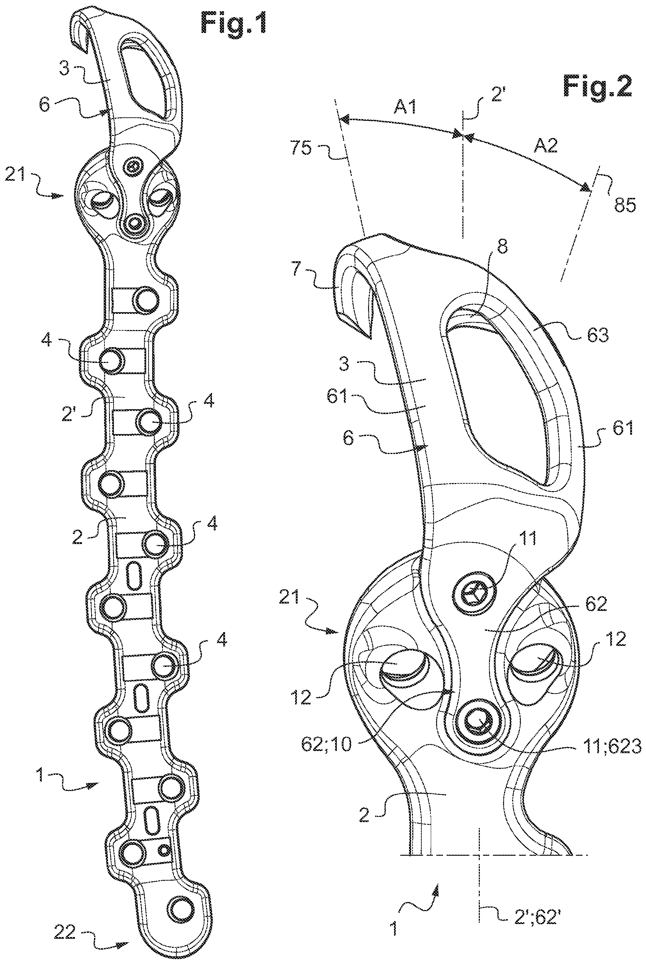

La plaque d'ostéosynthèse 1, illustrée de manière générale sur les figures 1 à 3, comporte deux parties :
- une partie longitudinale 2, destinée à recouvrir et à épouser le profil de la face externe du corps S du fémur F, et
- une partie d'extrémité supérieure 3, destinée à se positionner sur le grand trochanter T du fémur F.

Ces deux parties 2, 3 de la plaque d'ostéosynthèse 1 sont ici constituées par deux pièces distinctes assemblables/dissociables, qui sont décrites plus en détails ci-dessous.

De manière alternative, cette plaque d'ostéosynthèse 1 pourrait également être réalisée monobloc, dans une seule et même pièce.

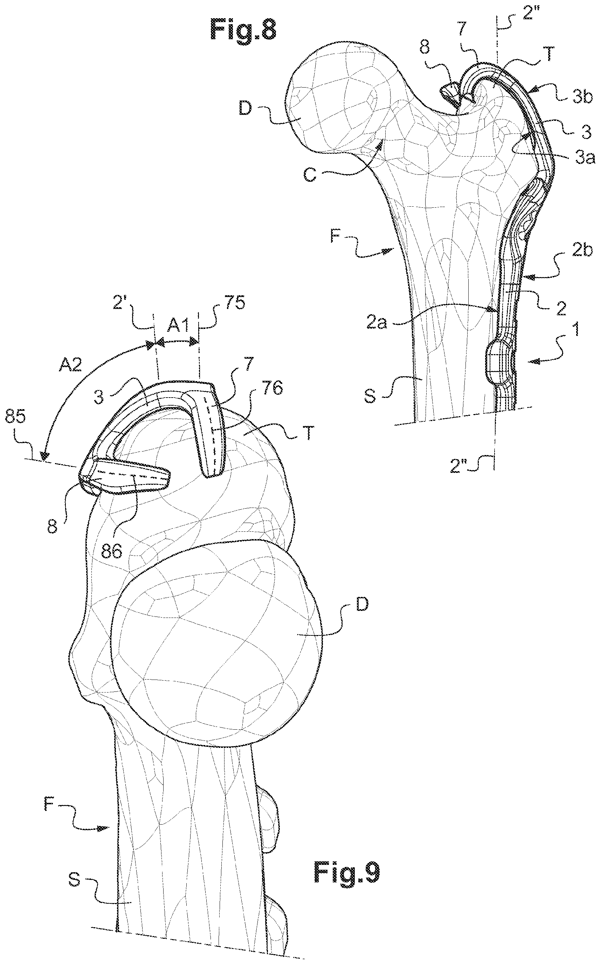

Chacune de ces deux parties 2, 3 comporte deux faces opposées visibles sur la figure 8:
- une face de dessous 2a, 3a, destinée à recouvrir l'os F, et
- une face de dessus 2b, 3b, destinée à venir à l'opposé de l'os F.

La partie longitudinale 2 consiste en une première pièce qui se présente sous la forme d'une platine ou barrette longitudinale.

Cette partie longitudinale 2 est munie d'une pluralité d'orifices traversants 4, répartis sur sa longueur, pour le positionnement d'éléments de fixation 5 dans le matériau osseux de réception (par exemple des vis dont seule la tête est visible sur la figure 7).

Tel que représenté sur la figure 1, cette partie longitudinale 2 se termine par deux extrémités, l'une supérieure 21 portant la partie d'extrémité supérieure 3 et l'autre inférieure 22 située à l'opposé.

Cette partie longitudinale 2 de la plaque d'ostéosynthèse 1 définit un axe longitudinal 2'.

Cet axe longitudinal 2' correspond avantageusement à une ligne médiane passant par les deux extrémités 21, 22 de cette partie longitudinale 2 ; cet axe longitudinal 2' correspond encore à un axe de symétrie passant par son extrémité supérieure 21.

Ici, les orifices traversants 4 sont disposés en quinconce de chaque côté de l'axe longitudinal 2', ménagés dans des excroissances latérales locales.

La face de dessous 2a de la partie longitudinale 2 définit encore un plan général 2", illustré sur la figure 8.

La partie d'extrémité supérieure 3 consiste en une seconde pièce, destinée à coopérer avec le grand trochanter.

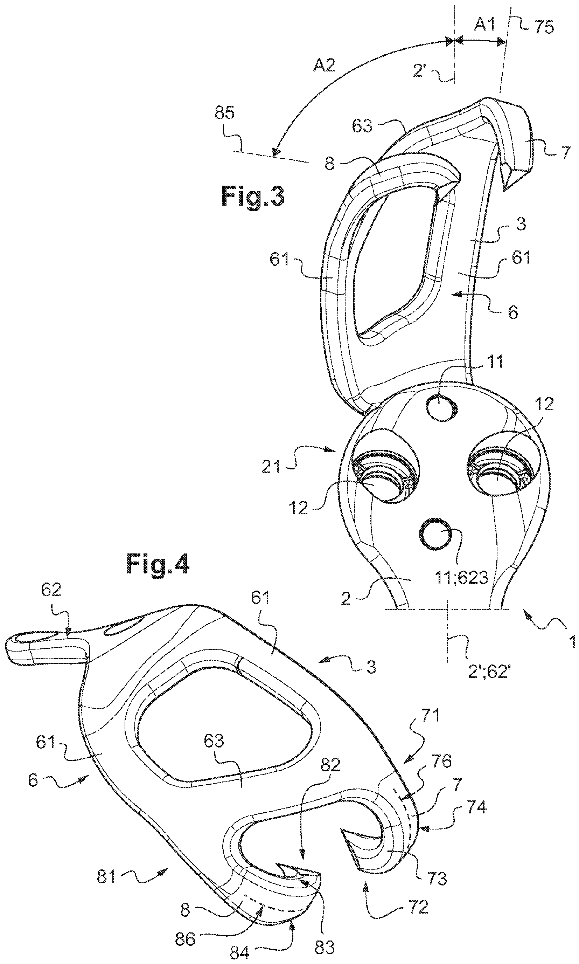

Cette partie d'extrémité supérieure 3 comporte une base 6 qui est solidarisée avec l'extrémité supérieure 21 de la partie longitudinale 2 (figures 1 à 3) et qui comporte deux branches 7, 8 formant crochets (figures 3 à 5).

Dans la présente description, les termes « branches » et « crochets » sont utilisés indifféremment pour dénommer les mêmes structures.

Les deux crochets 7, 8 consistent chacun en une tige recourbée, destinés à venir chacun épouser une partie de la surface périphérique du grand trochanter T.

Pour cela, ces deux crochets 7, 8 sont recourbés du côté de la face de dessous 2a de la partie longitudinale inférieure 2 et du côté de la face de dessous 3a de la partie d'extrémité supérieure 3.

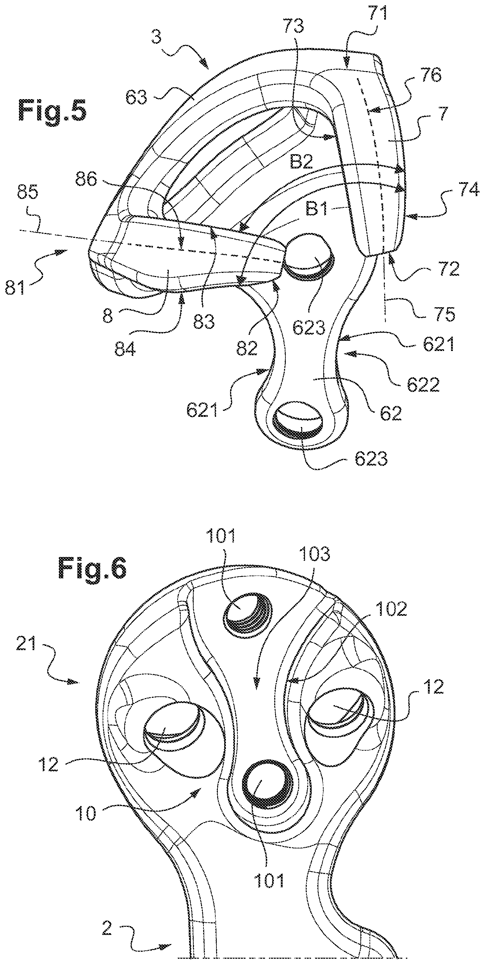

Les crochets 7, 8 présentent chacun deux extrémités reliées par deux bordures longitudinales (figures 4 et 5), à savoir :
- une extrémité arrière 71, 81, raccordée à la base 6,
- une extrémité avant 72, 82 libre, située à distance de la base 6 et présentant une forme acérée pour venir s'insérer dans le matériau osseux de réception,
- une bordure intérieure 73, 83, s'étendant en regard de l'autre crochet 7, 8, et
- une bordure extérieure opposée 74, 84.

Ces deux crochets 7, 8 comportent chacun un plan médian 75, 85 qui est défini et qui passe par leurs lignes courbes médianes 76, 86 respectives.

Les plans médians 75, 85 sont illustrés sous la forme d'un axe sur les différentes figures, par souci de simplification.
Cet axe correspond avantageusement à la ligne d'intersection entre un plan médian 75, 85 et le plan général 2" passant par la face de dessous 2a de la partie longitudinale 2.

La ligne courbe médiane 76, 86 de chaque crochet 7, 8 s'étend entre ses deux extrémités 71, 72 ou 81, 82, cela au moins approximativement au milieu de sa largeur délimitée par ses deux bordures 73, 74 ou 83, 84.

Les crochets 7, 8 sont orientés pour optimiser le transfert des efforts de compression, et ainsi pour favoriser la stabilité du foyer de fracture osseuse par l'utilisation des efforts musculaires.

Pour générer ce phénomène avantageux, ces deux crochets 7 et 8 sont agencés de sorte que :
- un premier crochet 7, dit « axial », est destiné à recouvrir la partie latérale du grand trochanter T, et
- un second crochet 8, dit « désaxé » ou « latéral », est destiné à recouvrir la partie postérieure du grand trochanter T.

A cet effet, comme on peut l'observer en particulier sur les figures 2, 3 et 9, les branches 7, 8 sont orientées de sorte que :
(i) le plan médian 75 de la première branche 7 définit un angle maximal A1 de 15°, en l'occurrence un angle de 13°, par rapport à l'axe bngitudinal 2' de la partie longitudinale 2, et
(ii) le plan médian 85 de la seconde branche 8 définit un angle A2 compris entre 50° et 100°, de préférence un angle compris entre 70° et 90°, en l'occurrence un angle de 72°, par rapport à l'axe longitudinal 2' de la partie longitudinale inférieure 2.

En d'autres termes, l'angle défini entre les plans médians 75 et 85 de ces deux branches 7, 8 est avantageusement compris entre 40° et 90°, de préférence compris entre 70° et 90°, en l'occurrence de l'ordre de 85°.

Les deux branches 7 et 8 se situent ici de part et d'autre de l'axe longitudinal 2'.

Vu du côté de la face de dessous 3a de la partie d'extrémité supérieure 3, la première branche 7 est agencée selon un angle horaire et la seconde branche 8 est agencée selon un angle antihoraire.

Les plans médians 75 et 85 de ces deux branches 7, 8 s'étendent en plus perpendiculairement, ou au moins approximativement perpendiculairement, par rapport au plan général 2" passant par la face de dessous 2a de la partie longitudinale 2.

En outre, partant de la bordure longitudinale extérieure 74 de la première branche 7 comme référentiel, la bordure longitudinale extérieure 84 de la seconde branche 8 définit un angle B1 compris entre 80° et 100°, en l'occurrencede 90° ; de la même manière, partant de la bordure longitudinale extérieure 74 de la première branche 7 comme référentiel, la bordure longitudinale intérieure 83 de cette seconde branche 8 définit quant à elle un angle B2 compris entre 60° et 80°, en l'occurrence de 70° (voir figure 5).

Cette caractéristique structurelle permet une mise en place du crochet sans risque de conflit avec le complexe musculo-tendineux et avec les autres parties molles de cette région anatomique, tout en optimisant son rôle mécanique dans la génération d'un effet « hauban ».

Les crochets 7, 8 ont avantageusement une largeur (correspondant à la distance séparant leurs bordures intérieure 73, 83 et extérieure 74, 84) comprise entre 3 et 5 mm.

La base 6 est quant à elle destinée à s'étendre sur la partie proximale du grand trochanter T.

Comme représenté sur les figures 4 et 5, la base 6 comporte deux bras 61 qui sont, d'un côté inférieur, raccordés à un tronçon saillant 62 pour sa solidarisation avec la partie longitudinale 2 et, d'un côté supérieur, raccordés par une traverse 63 et prolongés chacun par l'une des deux branches 7, 8 formant crochets.

Le tronçon saillant 62 constitue une partie des moyens pour la solidarisation de la partie d'extrémité supérieure 3 de manière amovible, et dissociable, sur la partie longitudinale 2.

Ce tronçon saillant 62, visible en particulier sur la figure 5, se présente sous la forme générale d'une tige délimitée par deux bordures latérales 621.

Les deux bordures latérales 621 présentent chacune une forme générale courbe concave et s'ouvrent à l'opposé l'une de l'autre, pour former un rétreint local 622.

Ce tronçon saillant 62 est encore muni de deux orifices 623, ménagés de part et d'autre du rétreint local.

Ce tronçon saillant 62 comporte ainsi un axe longitudinal 62' qui passe par ses deux orifices 623 et qui est ici destiné à s'étendre coaxialement à l'axe longitudinal 2' de la partie longitudinale 2 (figure 2).

Pour la réception de cette partie d'extrémité supérieure 3, tel qu'illustré sur la figure 6, l'extrémité supérieure 21 de la partie longitudinale 2 comporte une empreinte 10 dont le contour est complémentaire de celui du tronçon saillant 62 de la partie d'extrémité supérieure 3.

Plus précisément, cette empreinte complémentaire 10 comporte également deux orifices 101, destinés à être alignés avec les orifices 623 de la partie d'extrémité supérieure 3 pour la réception d'éléments rapportés de solidarisation entre eux, en l'occurrence deux vis 11 (dont seule leur tête est visible sur la figure 2).

Cette empreinte 10 s'ouvre au travers de la face de dessus 2b de la partie longitudinale 2, et aussi du côté de son extrémité supérieure 21.

Deux bordures longitudinales en regard 102, délimitant latéralement cette empreinte 10, sont convexes l'une en direction de l'autre pour définir un rétreint local 103, complémentaire de celui du tronçon saillant 62.

Les formes complémentaires de ce tronçon saillant 62 et de cette empreinte 10, avec les deux retreints locaux 622, 103, permettent, lorsque les deux parties 2 et 3 sont assemblées, d'optimiser leur positionnement relatif et aussi de renforcer le verrouillage mécanique en translation des pièces associées 2 et 3, selon l'axe longitudinal 2' de la partie longitudinale 2.

L'extrémité supérieure 21 de la partie longitudinale 2 comporte encore deux orifices traversants 12 qui sont ménagés de part et d'autre de l'empreinte 10, au niveau de son rétreint local 103.

Ces orifices traversants 12 sont prévus pour le positionnement de vis de fixation 13 dans le matériau osseux de réception (dont seule la tête est visible sur la figure 7).

Ces derniers éléments de fixation 13 de l'extrémité supérieure 21 de la partie longitudinale 2 ont pour intérêt de compléter la synthèse du fragment osseux proximal.

En pratique, la plaque d'ostéosynthèse 1 est tout d'abord assemblée par le montage de la partie d'extrémité supérieure 3 sur la partie longitudinale 2.

Cet assemblage est réalisé par le positionnement du tronçon saillant 62 de la partie d'extrémité supérieure 3 dans l'empreinte complémentaire 10 ménagée sur la partie longitudinale 2.

Cet assemblage est verrouillé par le positionnement des vis 11 dédiées.

Ces vis 11 sont destinées à s'étendre uniquement dans l'épaisseur de la plaque d'ostéosynthèse 1 (c'est-à-dire sans faire saillie notamment au niveau de la face inférieure 2a de la partie longitudinale 2).

Cette approche a en particulier l'intérêt de permettre un choix optimal des deux pièces destinées à constituer la partie longitudinale 2 et la partie d'extrémité supérieure 3, cela notamment pour tenir compte de la morphologie du grand trochanter T à équiper.

Ce montage réalisé, la plaque d'ostéosynthèse 1 est convenablement rapportée par le praticien sur le fémur F à équiper, avec :
- la partie longitudinale 2 positionnée de sorte à recouvrir la face externe du corps S du fémur F, et
- la partie d'extrémité supérieure 3 positionnée sur le grand trochanter T de ce fémur F.

Comme décrit précédemment en relation avec les figures 7 à 9, la première branche 7 est destinée à recouvrir la partie latérale du grand trochanter, et la seconde branche 8 est destinée à recouvrir la partie postérieure du grand trochanter.

Une fois la plaque d'ostéosynthèse 1 convenablement positionnée sur l'os, avec les extrémités acérées des crochets 7, 8 enfoncées dans l'os par impactages, il suffit au praticien de rapporter les différents éléments de fixation 5 et 13 dans le matériau osseux de réception au travers des orifices 4, 12 dédiés de la partie longitudinale 2.

De manière générale, cette plaque d'ostéosynthèse 1 est adaptée pour la réduction de fractures touchant le grand trochanter, en particulier les fractures proximales.

La mise en oeuvre d'une telle plaque d'ostéosynthèse 1 offre encore les avantages suivants : modularité, adaptation d'un crochet à l'anatomie du trochanter traité (gamme de tailles de crochets).

## Revendications

1. Plaque pour ostéosynthèse de l'extrémité supérieure d'un fémur, laquelle plaque (1) comporte deux parties :
- une partie longitudinale (2) dont une face de dessous (2a) est destinée à recouvrir la face externe du corps (S) du fémur (F), laquelle partie longitudinale (2) définit un axe longitudinal (2') et est munie de plusieurs orifices traversants (4, 12) pour le positionnement d'éléments de fixation (5, 13) dans le matériau osseux de réception, et
- une partie d'extrémité supérieure (3) dont une face de dessous (3a) est destinée à se positionner sur le grand trochanter (T) dudit fémur (F), laquelle partie d'extrémité supérieure (3) comporte au moins deux branches (7, 8) formant crochets qui sont recourbées du côté de la face de dessous (2a) de ladite partie longitudinale inférieure (2) et qui comportent chacune un plan médian (75, 85) défini par leur ligne courbe médiane (76, 86),
**caractérisée en ce que** lesdites branches (7, 8) sont agencées de sorte que :
(i) le plan médian (75) d'une première desdites branches (7) définit un angle (A1 ) maximal de 15° par rapport à l'axe longitudinal (2) de ladite partie longitudinale (2), et
(ii) le plan médian (85) d'une seconde desdites branches (8) définit un angle (A2) compris entre 50° et 110° par rapport à l'axe longitudinal (2') de ladite partie longitudinale (2).

2. Plaque selon la revendication 1, **caractérisée en ce que** les deux branches (7, 8) sont agencées de sorte que :
(i) le plan médian (75) d'une première desdites branches (7) définit un angle (A1 ) maximal de 15° par rapport à l'axe longitudinal (2) de ladite partie longitudinale (2), et
(ii) le plan médian (85) d'une seconde desdites branches (8) définit un angle (A2) compris entre 70° et 90° par rapport à l'axe longitudinal (2') de ladite partie longitudinale (2).

3. Plaque selon l'une quelconque des revendications 1 ou 2, **caractérisée en ce que** les plans médians (75, 85) respectifs des deux branches (7, 8) s'étendent perpendiculairement, ou au moins approximativement perpendiculairement, par rapport à un plan général (2") passant par la face de dessous (2a) de la partie longitudinale (2).

4. Plaque selon l'une quelconque des revendications 1 à 3, **caractérisée en ce que** les branches (7, 8) présentent chacune deux bordures longitudinales, une bordure intérieure (73, 83) en regard de l'autre branche (7, 8) et une bordure extérieure (74, 84) opposée, et **en ce que**, par rapport à la bordure longitudinale extérieure (74) de la première branche (7), la bordure longitudinale extérieure (84) de la seconde branche (8) définit un angle (B1) compris entre 80° et 100° et la bordure longitudinale intérieure (83) de la seconde branche (8) définit un angle (B2) compris entre 60° et 80°.

5. Plaque selon l'une quelconque des revendications 1 à 4, **caractérisée en ce que** la partie d'extrémité supérieure (3) comporte deux bras (61) qui sont, d'un côté inférieur, raccordés à la partie longitudinale (2) et, d'un côté supérieur, raccordés par une traverse (63) et prolongés chacun par l'une des branches (7, 8) formant crochets.

6. Plaque selon l'une quelconque des revendications 1 à 5, **caractérisée en ce que** la partie longitudinale (2) et la partie d'extrémité supérieure (3) sont dissociables, avec des moyens (62, 10) pour leur solidarisation amovible l'une avec l'autre.

7. Plaque selon la revendication 6, **caractérisée en ce que** la partie longitudinale (2) et la partie d'extrémité supérieure (3) comportent, pour l'une, un tronçon saillant (62) et, pour l'autre, une empreinte complémentaire (10), lequel tronçon saillant (62) et laquelle empreinte complémentaire (10) comportent des orifices traversants (623, 101) destinés à être alignés pour la réception d'éléments rapportés (11) de solidarisation entre eux.

8. Plaque selon la revendication 7, **caractérisée en ce que** le tronçon saillant (62) et l'empreinte complémentaire (10) sont tous deux délimités latéralement par deux bordures longitudinales (621, 102) en regard définissant un retreint local (622, 103), pour assurer notamment un verrouillage mécanique en translation selon l'axe longitudinal (2') de la partie longitudinale (2).

9. Plaque selon la revendication 8, **caractérisée en ce que** le tronçon saillant (62) et l'empreinte complémentaire (10) comportent deux orifices traversants (623, 101) pour le positionnement des éléments de solidarisation (11), ménagés de part et d'autre du retreint local (622, 103).

10. Plaque selon l'une quelconque des revendications 8 ou 9, **caractérisée en ce que** la partie longitudinale (2) ou la partie d'extrémité supérieure (3), munie de l'empreinte (10), comporte deux orifices traversants (12) ménagés de part et d'autre du retreint local (103), pour le positionnement d'éléments de fixation (13) dans le matériau osseux de réception.

11. Plaque selon l'une quelconque des revendications 7 à 10, **caractérisée en ce que** la partie d'extrémité supérieure (3) comporte le tronçon saillant (62), et **en ce que** la partie longitudinale (2) comporte l'empreinte complémentaire (10).

12. Dispositif d'ostéosynthèse comprenant (i) une plaque d'ostéosynthèse (1) selon l'une quelconque des revendications 1 à 11, et (ii) les éléments (5, 12) de fixation de ladite plaque d'ostéosynthèse (1) sur le matériau osseux de réception.

## Patentansprüche

1. Platte zur Osteosynthese des oberen Endes eines Oberschenkelknochens, wobei die Platte (1) zwei Teile aufweist:
- ein längs gerichtetes Teil (2), von dem eine Unterseite (2a) dazu bestimmt ist, die Außenseite des Körpers (S) des Oberschenkelknochens (F) abzudecken, wobei das längs gerichtete Teil (2) eine Längsachse (2') definiert und mit mehreren Durchgangslöchern (4, 12) zum Positionieren von Befestigungselementen (5, 13) im aufnehmenden Knochenmaterial versehen ist, und
- ein Teil (3) für das obere Ende, von dem eine Unterseite (3a) dazu bestimmt ist, sich auf dem Trochanter major (T) des Oberschenkelknochens (F) zu positionieren, wobei das Teil (3) für das obere Ende wenigstens zwei Haken bildende Zweige (7, 8) aufweist, die zur Unterseite (2a) des unteren längs gerichteten Teils (2) hin gebogen sind und von denen jeder eine durch deren Krümmungslinie (76, 86) definierte Mittelebene (75, 85) aufweist, **dadurch gekennzeichnet, daß** die Zweige (7, 8) derart angeordnet sind, daß
i) die Mittelebene (75) eines ersten (7) der besagten Zweige einen Winkel (A1) von maximal 15° gegenüber der Längsachse (2') des längs gerichteten Teils (2) definiert und
ii) die Mittelebene (85) eines zweiten (8) der besagten Zweige einen Winkel (A2) zwischen 50° und 110° gegenüber der Längsachse (2') des längs gerichteten Teils (2) definiert.

2. Platte gemäß Anspruch 1, **dadurch gekennzeichnet, daß** die beiden Zweige (7, 8) derart angeordnet sind, daß
i) die Mittelebene (75) eines ersten (7) der besagten Zweige einen Winkel (A1) von maximal 15° gegenüber der Längsachse (2') des längs gerichteten Teils (2) definiert und
ii) die Mittelebene (85) eines zweiten (8) der besagten Zweige einen Winkel (A2) zwischen 70° und 90° gegenüber der Längsachse (2') des längs gerichteten Teils (2) definiert.

3. Platte gemäß Anspruch 1 oder 2, **dadurch gekennzeichnet, daß** sich die jeweiligen Mittelebenen (75, 85) der beiden Zweige (7, 8) senkrecht oder wenigstens näherungsweise senkrecht zu einer durch die Unterseite (2a) des längs gerichteten Teils (2) gehenden allgemeinen Ebene (2") erstrecken.

4. Platte gemäß einem der Ansprüche 1 bis 3, **dadurch gekennzeichnet, daß** jeder der beiden Zweige (7, 8) zwei Längsränder, einen inneren, zum anderen Zweig hin gerichteten Rand (73, 83) und einen entgegengesetzt angeordneten äußeren Rand (74, 84), aufweist und daß, in Bezug auf den äußeren Längsrand (74) des ersten Zweiges (7), der äußere Längsrand (84) des zweiten Zweiges (8) einen zwischen 80° und 100° betragenden Winkel (B1) und der innere Längsrand (83) des zweiten Zweiges (8) einen zwischen 60° und 80° betragenden Winkel (B2) definiert.

5. Platte gemäß einem der Ansprüche 1 bis 4, **dadurch gekennzeichnet, daß** das Teil (3) für das obere Ende zwei Arme (61) aufweist, die an einer unteren Seite mit dem längs gerichteten Teil (2) verbunden sind und an einer oberen Seite durch eine Querstrebe (63) verbunden und jeweils durch einen der Haken bildenden Zweige (7, 8) verlängert sind.

6. Platte gemäß einem der Ansprüche 1 bis 5, **dadurch gekennzeichnet, daß** das längs gerichtete Teil (2) und das Teil (3) für das obere Ende voneinander trennbar sind, und zwar durch Mittel (62, 10) für deren trennbares miteinander Verbinden.

7. Platte gemäß Anspruch 6, **dadurch gekennzeichnet, daß** vom längs gerichteten Teil (2) und vom Teil (3) für das obere Ende das eine einen hervorstehenden Abschnitt (62) und das andere eine komplementäre Einbuchtung (10) aufweist, die Durchgangslöcher (623, 101) aufweisen, die dazu bestimmt sind, zur Aufnahme zugefügter Befestigungselemente (11) untereinander ausgerichtet zu sein.

8. Platte gemäß Anspruch 7, dadurch gekenntzeichnet, daß der hervorstehende Abschnitt (62) und die komplementäre Einbuchtung (10) beide seitlich durch zwei entgegengesetzt angeordnete Längsränder (621, 102) begrenzt sind, die eine örtliche Vertiefung (622, 103) definieren, um eine mechanische translatorische Verriegelung entlang der Längsachse (2') des längs gerichteten Teils (2) sicherzustellen.

9. Platte gemäß Anspruch 8, **dadurch gekennzeichnet, daß** der hervorstehende Abschnitt (62) und die komplementäre Einbuchtung (10) zwei Durchgangslöcher (623, 101) zum Positionieren der beiderseits der örtlichen Vertiefung (622, 103) angeordneten Befestigungselemente (11) aufweisen.

10. Platte gemäß einem der Ansprüche 8 oder 9, **dadurch gekennzeichnet, daß** das längs gerichtete Teil (2) bzw. das Teil (3) für das obere Ende, das mit der Einbuchtung (10) versehen ist, zwei beiderseits der örtlichen Vertiefung (103) angeordnete Durchgangslöcher (12) für das Positionieren von Befestigungselementen (13) im aufnehmenden Knochenmaterial aufweist.

11. Platte gemäß einem der Ansprüche 7 bis 10, **dadurch gekennzeichnet, daß** das Teil (3) für das obere Ende den hervorstehenden Abschnitt (62) aufweist und daß das längs gerichtete Teil (2) die komplementäre Einbuchtung (10) aufweist.

12. Osteosynthesevorrichtung mit (i) einer Platte (1) zur Osteosynthese gemäß einem der Ansprüche 1 bis 11 und (ii) mit Elementen (5, 12) zum Befestigen der Platte (1) zur Osteosynthese an dem aufnehmenden Knochenmaterial.

## Claims

1. A plate for osteosynthesis of the upper end of a femur, wherein said plate (1) includes two parts:
- a longitudinal part (2), a bottom face (2a) of which is intended to cover the external face of the body (S) of the femur (F), said longitudinal part (2) defining a longitudinal axis (2') and being provided with several through-orifices (4, 12) for the positioning of fastening elements (5, 13) in the receiving bone material, and
- an upper end part (3), a bottom face (3a) of which is intended to be positioned on the greater trochanter (T) of said femur (F), said upper end part (3) including at least two hook branches (7, 8) that are curved towards the bottom face (2a) of said lower longitudinal part (2) and that each include a median plane (75, 85) defined by their median curved line (76, 86),
**characterized in that** said branches (7, 8) are arranged so that:
(i) the median plane (75) of a first of said branches (7) defines a maximum angle (A1) of 15° with respect to the longitudinal axis (2) of said longitudinal part (2), and
(ii) the median plane (85) of a second of said branches (8) defines an angle (A2) comprised between 50° and 110° with respect to the longitudinal axis (2') of said longitudinal part (2).

2. The plate according to claim 1, **characterized in that** the two branches (7, 8) are arranged so that:
(i) the median plane (75) of a first of said branches (7) defines a maximum angle (A1) of 15° with respect to the longitudinal axis (2) of said longitudinal part (2), and
(ii) the median plane (85) of a second of said branches (8) defines an angle (A2) comprised between 70° and 90° with respect to the longitudinal axis (2') of said longitudinal part (2).

3. The plate according to any one of claims 1 or 2, **characterized in that** the respective median planes (75, 85) of the two branches (7, 8) extend perpendicularly, or at least approximately perpendicularly, with respect to a general plane (2") passing through the bottom face (2a) of the longitudinal part (2).

4. The plate according to any one of claims 1 to 3, **characterized in that** the branches (7, 8) each have two longitudinal edges, an inner edge (73, 83) opposite the other branch (7, 8) and an opposite outer edge (74, 84), and **in that**, with respect to the outer longitudinal edge (74) of the first branch (7), the outer longitudinal edge (84) of the second branch (8) defines an angle (B1) comprised between 80° and 100° and the inner longitudinal edge (83) of the second branch (8) defines an angle (B2) comprised between 60° and 80°.

5. The plate according to any one of claims 1 to 4, **characterized in that** the upper end part (3) includes two arms (61) that are, on a lower side, connected to the longitudinal part (2) and, on an upper side, connected by a crossbar (63) and each extended by one of the hook branches (7, 8).

6. The plate according to any one of claims 1 to 5, **characterized in that** the longitudinal part (2) and the upper end part (3) are separable from each other, with means (62, 10) for the removable attachment thereof to each other.

7. The plate according to claim 6, **characterized in that** the longitudinal part (2) and the upper end part (3) include, for the one, a protruding section (62) and, for the other one, a complementary cavity (10), wherein said protruding section (62) an said complementary cavity (10) include through-orifices (623, 101) intended to be aligned for receiving added elements (11) for the fastening thereof to each other.

8. The plate according to claim 7, **characterized in that** the protruding section (62) and the complementary cavity (10) are both delimited laterally by two opposite longitudinal edges (621, 102) defining a local necking (622, 103), to provide in particular a mechanical locking in translation along the longitudinal axis (2') of the longitudinal part (2).

9. The plate according to claim 8, **characterized in that** the protruding section (62) and the complementary cavity (10) include two through-orifices (623, 101) for the positioning of the fastening elements (11), arranged on either side of the local necking (622, 103).

10. The plate according to any one of claims 8 or 9, **characterized in that** the longitudinal part (2) or the upper end part (3), provided with the cavity (10), include two through-orifices (12) arranged on either side of the local necking (103), for the positioning of fastening elements (13) in the receiving bone material.

11. The plate according to any one of claims 7 to 10, **characterized in that** the upper end part (3) includes the protruding section (62), and **in that** the longitudinal part (2) includes the complementary cavity (10).

12. An osteosynthesis device comprising (i) an osteosynthesis plate (1) according to any one of claims 1 to 11, and (ii) the elements (5, 12) for the fastening of said osteosynthesis plate (1) to the receiving bone material.
